Europäisches Patentamt

European Patent Office

Office européen des brevets

(19)

(11) Numéro de publication: **0 424 518 B1**

(12) **FASCICULE DE BREVET EUROPEEN**

(45) Date de publication de fascicule du brevet: **21.12.94**

(51) Int. Cl.5: **A61K 39/10**, A61K 37/56, //C12N9/88

(21) Numéro de dépôt: **90908234.9**

(22) Date de dépôt: **11.05.90**

(86) Numéro de dépôt internationale : **PCT/FR90/00337**

(87) Numéro de publication internationale : **WO 90/13312 (15.11.90 90/26)**

(54) **UTILISATION D'ADENYL CYCLASE COMME VACCIN CONTRE BORDETELLA.**

(30) Priorité: **12.05.89 FR 8906321**

(43) Date de publication de la demande: **02.05.91 Bulletin 91/18**

(45) Mention de la délivrance du brevet: **21.12.94 Bulletin 94/51**

(84) Etats contractants désignés: **AT BE CH DE DK ES FR GB IT LI LU NL SE**

(56) Documents cités:
EP-A- 0 267 998
EP-A- 0 272 174
EP-A- 0 301 954
EP-A- 0 338 169
WO-A-87/03301

PROCEEDINGS OF THE 4th INTERNATL. SYMPOSIUM ON PERTUSSIS, JointIABS/WHO Meeting, Genève (CH), 1984, Develop. BIOL. Standard, vol. 61, S. Karger (Bale, CH); P. NOVOTNY et al., pp. 27-41&NUM;

(73) Titulaire: **INSTITUT PASTEUR**
**25-28, rue du Docteur Roux**
**F-75724 Paris Cédex 15 (FR)**

(72) Inventeur: **GUISO-MACLOUF, Nicole**
**13, boulevard Saint-Marcel**
**F-75013 Paris (FR)**

(74) Mandataire: **Gutmann, Ernest et al**
**Ernest Gutmann - Yves Plasseraud S.A.**
**3, rue Chauveau-Lagarde**
**F-75008 Paris (FR)**

Rank Xerox (UK) Business Services
(3.10/3.09/3.3.3)

INFECTION & IMMUNITY, vol. 50, no. 1, October 1985, American Society for Microbiology; P. NOVOTNY et al., pp. 199-206&NUM;

CHEMICAL ABSTRACTS, vol. 112, no. 19, 07 Mai 1990, Columbus, OH (US); N. GUISO et al., p. 538, no. 176554d&NUM;

JOURNAL OF BIOL. CHEMISTRY, vol. 251, 05 December 1986; p. 16264&NUM;

INFECTION & IMMUNITY, vol. 56, December 1988;, pp. 3189 and 3194&NUM;

PROCEEDINGS OF THE NATL. ACADEMY OF SCIENCES USA, vol. 86, May 1989; pp. 3554 and 3558&NUM;

INFECTION & IMMUNITY, vol. 58, February 1990; p. 352&NUM;

MICROBIAL PATHOG., vol. 7, 1989; p. 373&NUM;

## Description

L'invention concerne des vaccins capables de protéger l'homme ou l'animal contre les infections létales provoqées par les Bordetella. Elle vise en particulier l'utilisation de préparations vaccinantes élaborées à partir de Bordetella ou plus particulièrement d'adényl cyclase produite par ces bactéries en tant qu'antigènes protecteurs contre les effets toxiques des infections dues aux Bordetella.

On sait que les Bordetella, plus particulièrement Bordetella pertussis, Bordetella parapertussis et Bordetella bronchiseptica, sont responsables de pathologies respiratoires chez les vertébrés.

Ainsi, chez l'homme, B.pertussis est resposnable de la coqueluche, maladie infantile très répandue à travers le monde.

La vaccination contre la coqeluche est à ce jour le plus généralement réalisée à l'aide de bactéries entières inactivées.

Cependant, ces vaccins ne sont pas toujours dépourvus de toxicité étant donné que les facteurs de virulence sont constitués par les protéines sécrétées par les bactéries et non par ces bactéries elles-mêmes. Les protéines, même après la mort des bactéries, peuvent donc exercer des effets pathologiques graves.

Parmi les déterminants de virulence de B.pertussis, on citera les adhésines tels les agglutinogènes (AGG), l'hémagglutinine filamenteuse (FHA) et la toxine de pertussis (PTx), une toxine cytotrachéale (TCT), une toxine dermonécrotique (DNT) et une adényl cyclase-hémolysine (AC). Cette dernière est synthétisée sous forme d'un large précurseur de 1706 résidus. La partie aminoterminale de la molécule porte l'activité adényl cyclase et la partie carboxyterminale présente une forte homologie avec le produit du gène Hly d'E.coli (hémolysine). Cette adényl cyclase a la propriété d'être activée par la calmoduline.

La recherche de méthodes de prévention mieux adaptées a conduit les inventeurs à étudier le rôle de chacun des déterminants de virulence et à mettre au point un modèle d'infection expérimental mimant le processus de la maladie naturelle.

Ces travaux ont permis de constater le rôle de l'adényl cyclase en tant que cytotoxine et en tant qu'antigène protecteur et à développer une nouvelle utilisation des préparations vaccinantes à base d'adényl cyclase ou de préparations bactériennes produisant de l'adényl cyclase.

EP-A-0 272 174 décrit une méthode de purification d'adényl cyclase de Bordetella. Cette demande de brevet décrit également l'utilisation de suspensions bactériennes de différentes espèces de Bordetella pour obtenir une protection contre B.pertussis. L'utilisation d'adényl cyclase purifiée de B.bronchiseptica n'est pas décrite.

EP-A- 0 338 169 décrit une protection croisée entre B.pertussis et B.parapertussis, obtenue par l'administration d'adényl cyclase.

Novotny et al. (Develop. Biol. Stand. 61,27-41, 1984) décrit l'utilisation d'une protéine provenant de Bordetella pour obtenir une protection contre l'infection par B.pertussis. La protéine (P69) a été désignée comme "adényl cyclase". Ceci étant, des travaux ultérieurs ont démontré que la protéine n'était pas l'adényl cyclase mais une agglutinogène.

L'invention concerne plus spécialement l'utilisation d'une adényl cyclase provenant de Bordetella pertussis ou de Bordetella bronchiseptica ou d'un fragment actif de cette adényl cyclase, pour l'obtention d'un médicament destiné à conférer chez l'homme ou chez l'animal, une protection croisée contre les infections et les effets toxiques causés par B. bronchiseptica ou par B. pertussis, respectivement.

Selon un autre aspect, l'invention concerne B. bronchiseptica souche 9.73S déposée à la CNCM sous le n° I-858.

Dans un mode préféré de réalisation de l'invention, la préparation vaccinante est élaborée à partir d'adényl cyclase de B.bronchiseptica et est mise en oeuvre pour protéger contre les infections et les effets toxiques causés par B.pertussis.

On notera que cette protection croisée obtenue à partir de souches de B.bronchiseptica présente l'avantage d'utiliser des souches à croissance plus rapide que B.pertussis pour élaborer des vaccins contre les infections provoquées par B.pertussis.

Selon un autre mode de réalisation de l'invention, la préparation vaccinante est élaborée à partir d'adényl cyclase de B.pertussis et est mise en oeuvre pour protéger contre les infections et les effets toxiques causés par B.bronchiseptica.

L'adényl cyclase des Bordetella à laquelle il est fait référence ci-dessus est une adényl cyclase telle qu'obtenue par mise en contact d'un surnageant d'une culture bactérienne de Bordetella avec un gel Affigel-calmoduline®.

Il s'agit plus spécialement d'une préparation d'adényl cyclase telles que décrites dans la demande FR 2606789 déposée le 17.11.86.

On rappelle que ces préparations sont caractérisées en ce qu'elles possèdent une pureté élevée et sont pratiquement totalement dépourvues de produits contaminants bactériens, notamment de pertussis toxines, de lipopolysaccharide (ou LPS) et d'hémagglutinine filamenteuse (ou FHA).

L'adényl cyclase de ces préparations se présente sous une forme homogène sédimentant sur un gradient de densité en saccharose avec un coefficient S égal à 3,6. Elle existe sous deux formes moléculaires de 45 et 43 kDa respectivement, structurellement apparentées.

De telles préparations d'adényl cyclase possèdent une activité pouvant atteindre et même dépasser 1600 $\mu$mole de cAMP min$^{-1}$ mg$^{-1}$.

Ces préparations peuvent être élaborées à partir de cultures de bactéries exprimant l'AC (adényl cyclase), plus spécialement de bactéries pathogènes dont l'AC est capable d'interférer avec l'AC des cellules eucaryotes, par mise en contact d'un surnageant préalablement concentré de cultures de bactéries exprimant l'adénylate cyclase, ou d'un extrait de ces bactéries avec de la calmoduline.

Pour obtenir l'enzyme sous forme libre, on utilise de la calmoduline fixée à un support, puis on récupère l'enzyme adsorbée à l'aide d'un agent dénaturant qui est à son tour éliminé, et on recueille la préparation avec l'enzyme libre.

Le support est plus spécialement constitué par un matériau inerte vis-à-vis de la préparation renfermant l'enzyme, et capable de retenir les molécules de poids moléculaire éle vé, tel qu'un gel ou un matériau filtrant.

Le matériau filtrant est avantageusement en nitrocellulose ou en une matière plastique et présente une porosité de 0,45-0,22 $\mu$m.

L'agent dénaturant est de préférence de l'urée, de préférence 4 à 8,8 M.

Le surnageant concentré de la culture bactérienne est obtenu en soumettant un surnageant de cultures bactériennes exprimant l'AC, à une ou plusieurs opérations de filtrations, à l'aide de filtres de nitrocellulose ou de matière plastique de porosité avantageusement de 0,45 à 0,22 $\mu$m, puis, en incubant les filtres avec un agent détergent afin de libérer l'AC et en éliminant de la préparation d'AC les matériaux insolubles présents.

L'agent détergent est par exemple du Triton$^{®}$ ou du NP40$^{®}$.

L'extrait bactérien est obtenu par traitement des cellules bactériennes exprimant l'adénylate cyclase avec de l'urée et récupération du surnageant.

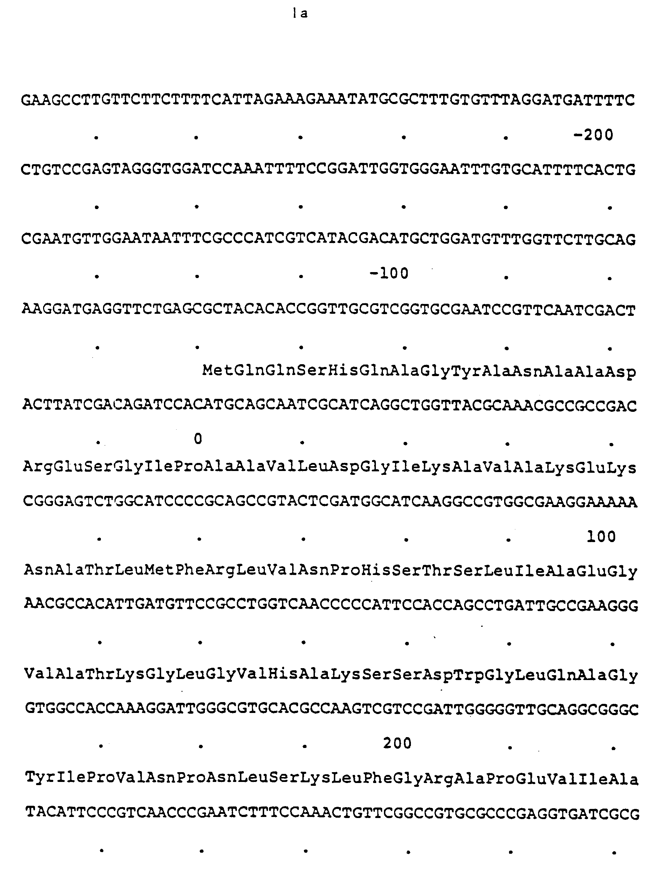

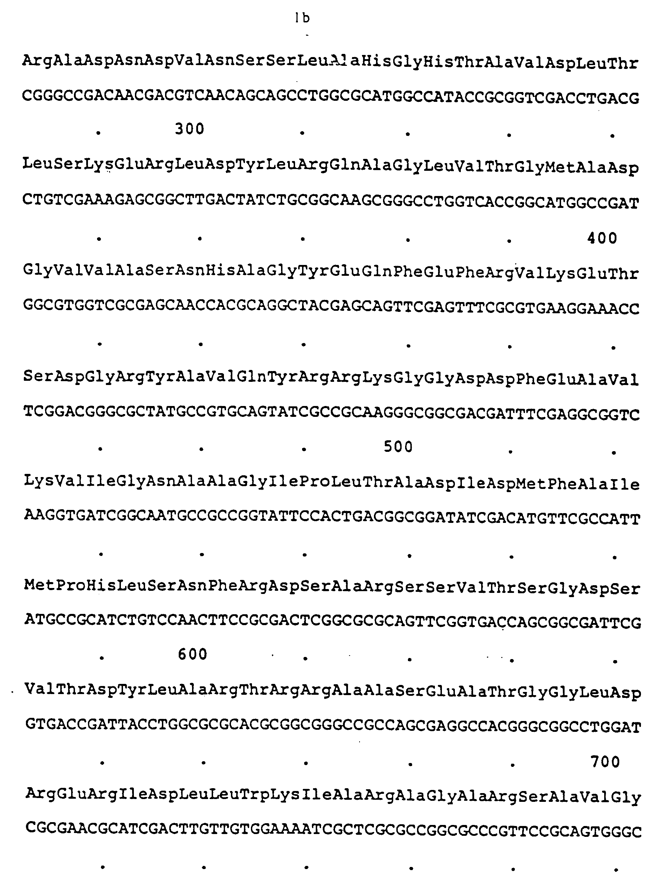

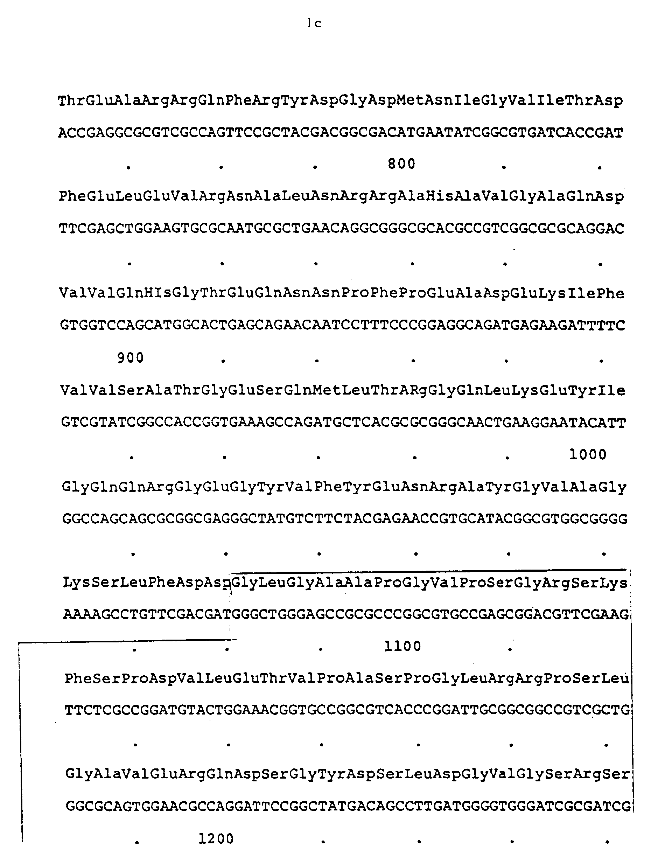

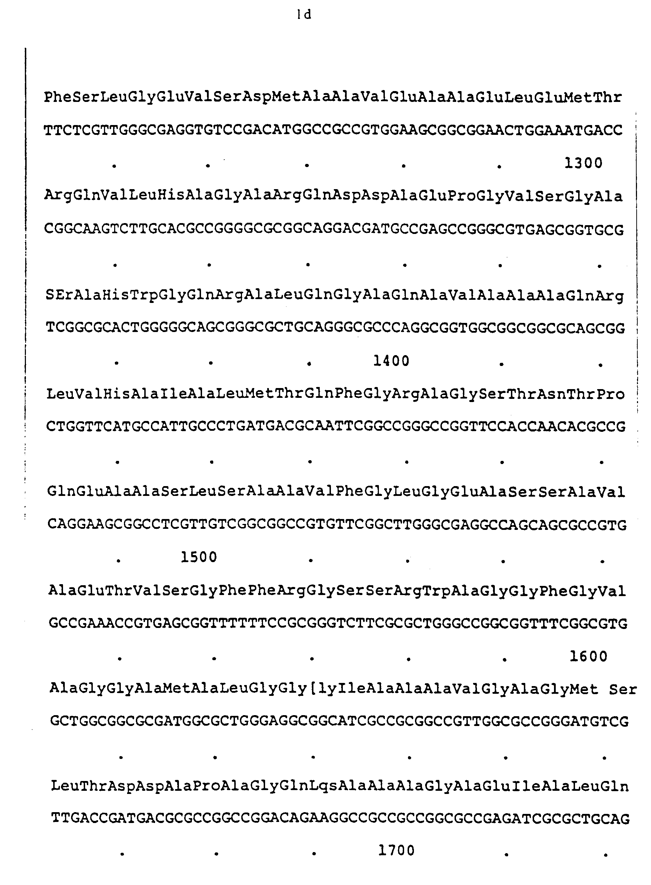

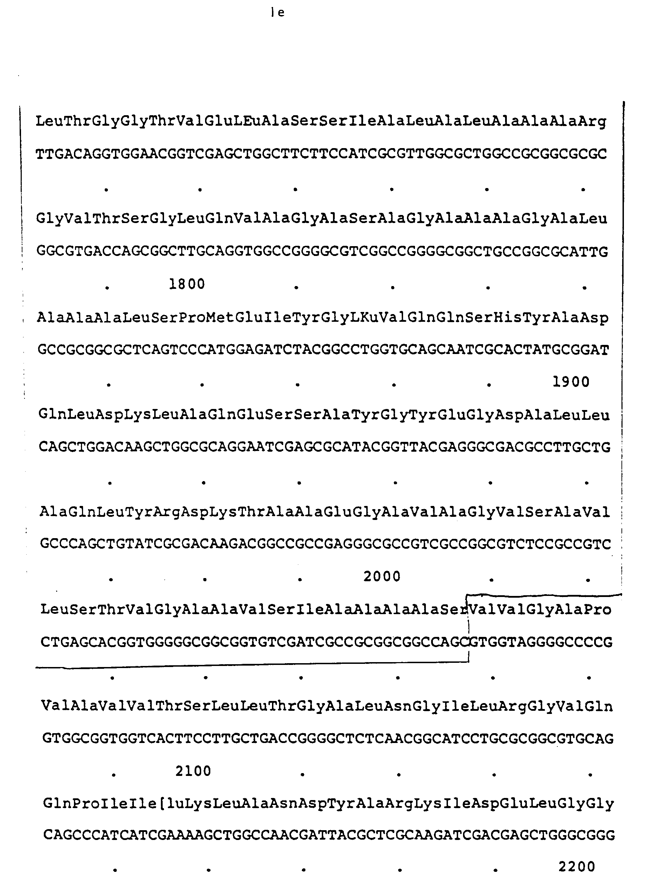

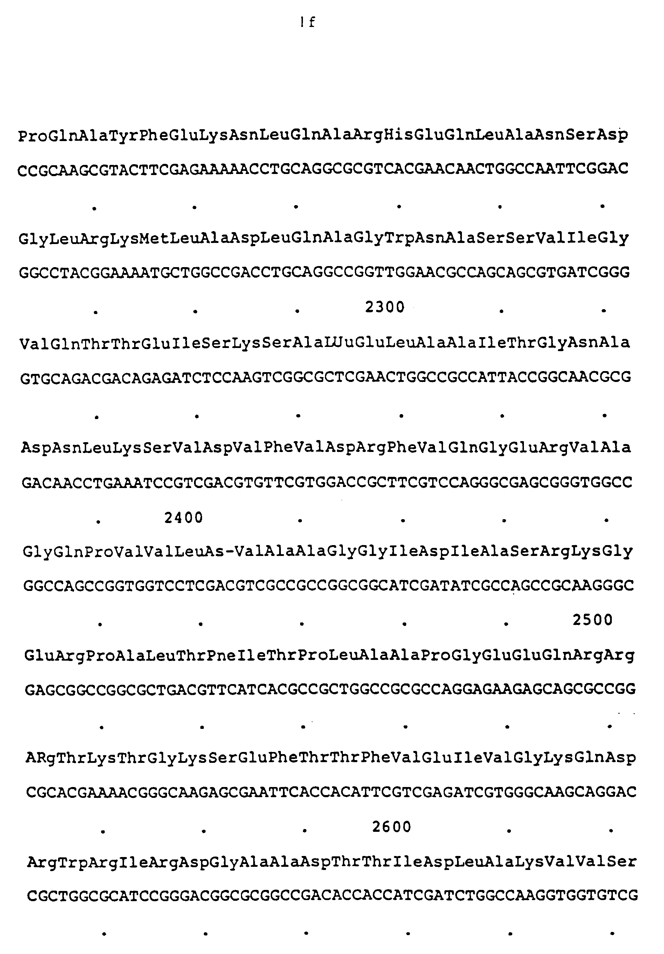

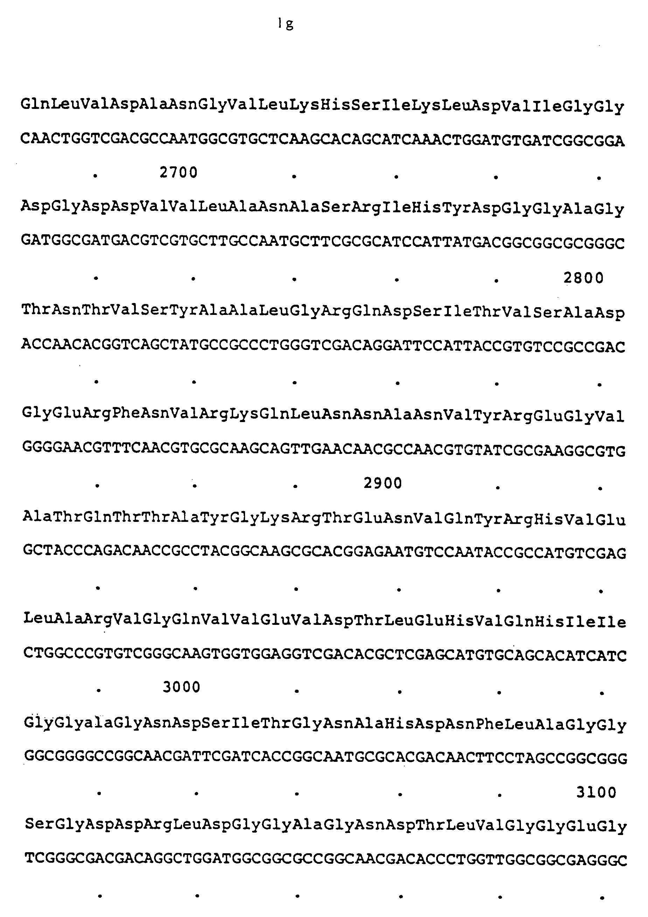

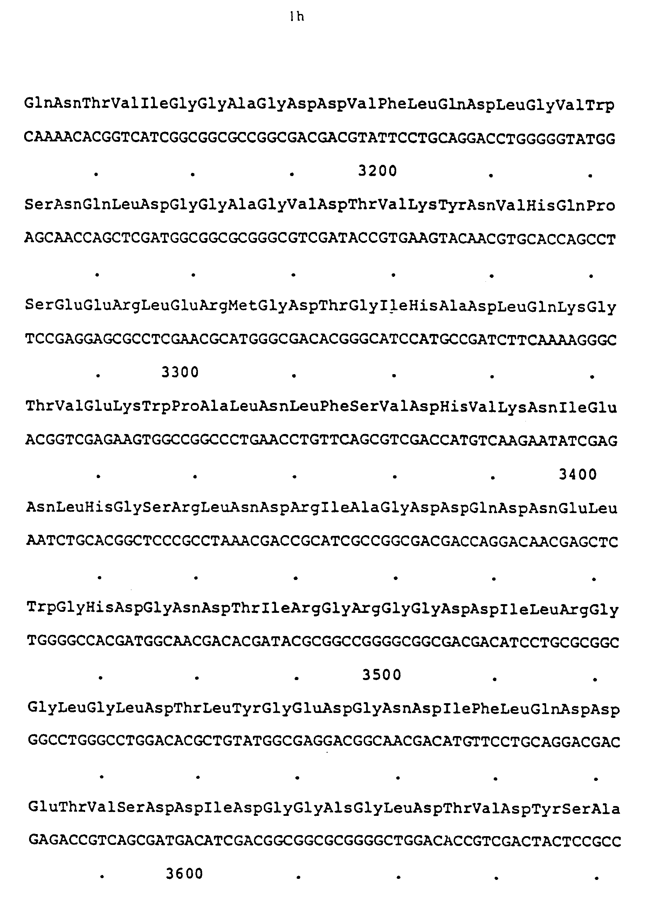

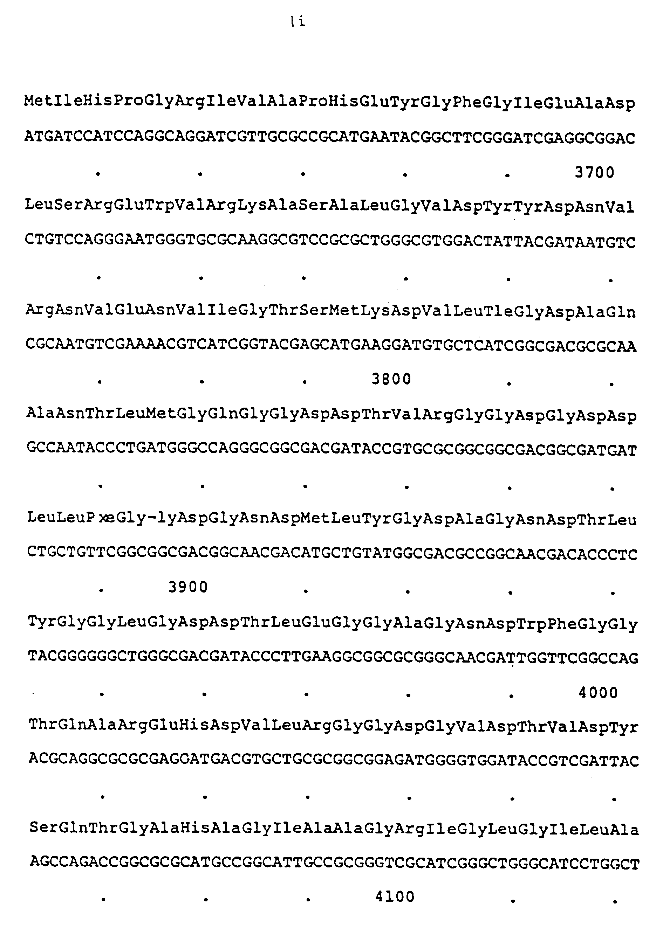

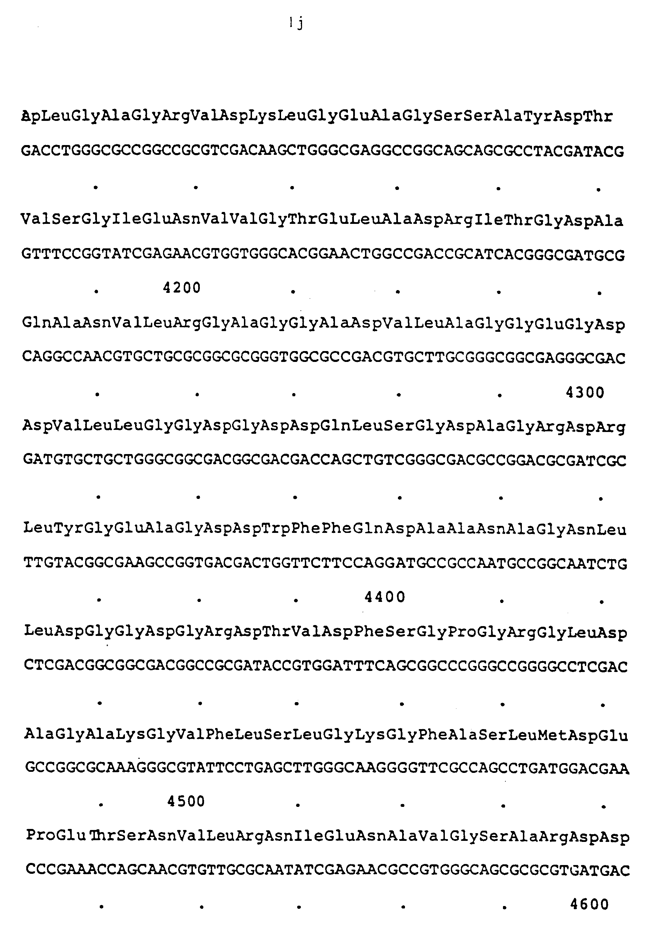

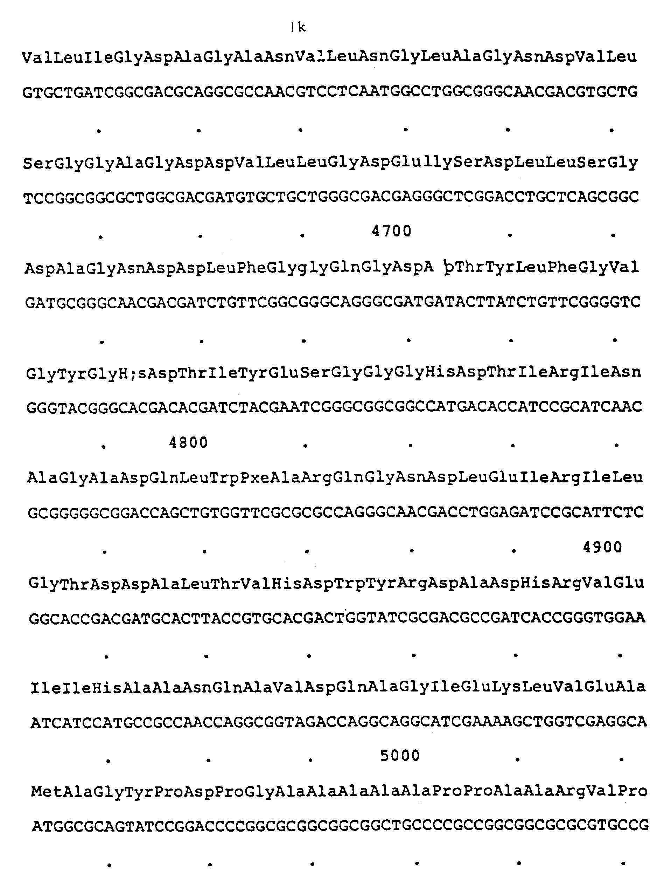

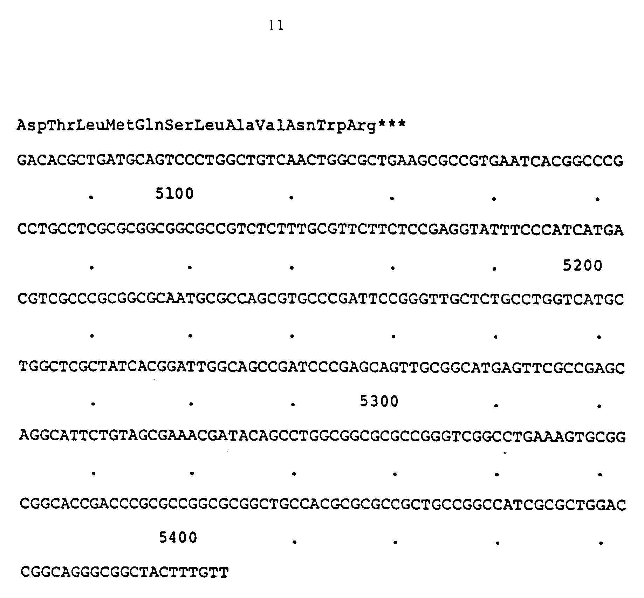

En variante, on utilise une adényl cyclase telle qu'exprimée par la séquence nucléotidique donnée sur la figure unique avec en correspondance la séquence d'acides aminés.

Il va de soi que les bases de la séquence nucléotidique considérée peuvent être dans un ordre différent de celui trouvé dans les gènes et/ou que ces bases peuvent être, le cas échéant, substituées dès lors qu'une sonde élaborée à partir d'une telle séquence donne une réponse caractéristique et non équivoque quant à la capacité de reconnaître la présence d'un gène codant pour une protéine à activité adénylate cyclase.

Toute séquence nucléotidique hybridable avec celle de l'enchaînement de cette séquence telle qu'obtenue par transcription enzymatique inverse de l'ARN correspondant ou encore par synthèse chimique entre également dans le cadre de l'invention.

Les préparations vaccinantes utilisées ci-dessus, peuvent être associées avec de la FHA et/ou de la PTx dans le même inoculum ou non.

Dans ce dernier cas, la FHA est administrée en même temps que la préparation vaccinante ou à un temps différent. Les préparations de FHA sont avantageusement obtenues selon, par exemple, la méthode de SATO et al dans Infect. Immun, 1983, 41, 310-320 ou celle d'IMAIZUMI et al dans Journal of Microbiol. Methods, 2, 334-347 (1984).

Selon un autre aspect, l'invention vise en tant que vaccins capables d'induire une protection contre les effets toxiques et les infections causées par les Bordetella, des vaccins moléculaires renfermant au moins la partie active de la séquence d'acides aminés représentée sur la figure unique, le cas échéant associée, dans le même inoculum ou non, à de la FHA et/ou de la PTx.

Ces vaccins sont utilisés conformément à l'invention pour effectuer des protections croisées.

Dans la mise en oeuvre de l'invention, les préparations vaccinantes à base d'adényl cyclase produite par ces bactéries, avantageusement purifiée, ainsi que les vaccins élaborés à partir d'adényl cyclase recombinante sont utilisés aux doses et sous les formes d'administration habituelle, en particulier sous les formes classiques administrables par voie intranasale, orale ou parentérale.

D'autres caractéristiques et avantages de l'invention apparaîtront dans la description des exemples qui suivent et en se reportant à la figure unique qui représente la séquence de nucléotide de la partie active du gène de B.pertussis codant pour l'adényl cyclase et la séquence correspondante d'acides aminés.

Le modèle d'infection respiratoire utilisé dans les essais rapportés dans les exemples est un modèle d'infection par voie intranasale. La sélection in vivo à partir d'un poumon de souris infectée par B.pertussis d'un dérivé hyperpathogène de la souche virulente de B.pertussis 18323S (souche de référence internationale pour les épreuves d'évaluation des vaccins), a permis d'observer chez la souris adulte, après injection des bactéries, par voie intranasale, une alvéolite oedémateuse hémorragique aigue (AOHA), létale en 48-72h.

Les analyses des produits des exemples sont effectuées comme suit.

L'activité de l'AC est mesurée selon la méthode de White A.A. dans Methods Enzymol., 38C, 41-46, 1974, telle que modfiée par Hanoune et al (J. Biol. Chem., 252, 2039-2046, 1977).

Les activités de PTx et de FHA sont mesurées selon la méthode de TUOMANON S. et WEISS A. dans J. Inf. Dis., 152, 118-125, 1985.

## EXEMPLE 1 : CULTURE DE B. BRONCHISEPTICA ET OBTENTION D'UN SURNAGEANT A CONCENTRATION ELEVEE EN ADENYLATE CYCLASE

On effectue une préculture de B.bronchiseptica 9.73, phase I et d'un variant avirulent, phase IV, spontanément, stable de cette souche, pendant 48 heures à 36°C, sur un milieu d'agar modifié Stainer-Scholte supplémenté avec de la cyclodextrine (selon IMAIZUIMI et al dans J. Chim. Microbiol., 17, 781-786, 1983). On transfère ensuite la préculture dans un milieu liquide modifié de Stainer-Scholte sans cyclodextrine. La souche de B.bronchiseptica 9.73 a été isolée à partir des nasaux d'un lièvre.

Les cultures liquides sont soumises à agitation à 150 t/min. pendant 15 heures à 36°C, dans un erlenmeyer de 1 litre contenant 250 ml de milieu. On poursuit la culture jusqu'à l'obtention d'une densité optique $DO_{650}$ = 1,2 ± 0,2. Les bactéries sont alors éliminées par centrifugation à 5000 x g durant 25 min.

Les surnageants de culture sont conservés à -30°C jusqu'à utilisation ou concentrés directement.

Aux fins de concentration, on filtre, sur des filtres de type Millipore[R] HAWP de 0,45 $\mu$m, 3 litres de surnageant de culture contenant environ 10 $\mu$g de protéines et 0,1 unité d'enzyme par ml. On retient ainsi plus de 90% de l'activité enzymatique sur les filtres. 80% environ de cette activité peuvent être récupérés par incubation des filtres dans 40 ml d'un tampon A constitué par du Tris.HCL, 50 mM, pH 8 contenant 6 mM de $MgCl_2$ et 0,1% de Triton X.100[R]. Le matériau insoluble est éliminé par centrifugation durant 30 min. à 15000 x g à 4°C. L'activité spécifique du surnageant de culture concentré est de 115 unités par milligramme de protéine.

Une suspension bactérienne de la souche 9.73 a été injectée par voie intranasale à une souris ; 48 heures après les poumons ont été prélevés et broyés et un clone hémolytique isolé, à savoir le clone 9.73S. Ce clone a été déposé le 12 mai 1989 à la CNCM sous le n° I-858. Ce clone est hémolytique, et synthétise de grandes quantités d'adényl cyclase. Une suspension bactérienne de ce clone provoque un oedème hémorragique au niveau du poumon chez la souris.

## EXEMPLE 2 : PROCEDE D'OBTENTION D'ADENYL CYCLASE EXTRACYTOPLASMIOUE PURIFEE SOUS FORME LIBRE

On ajoute 40 ml de surnageant de culture concentré à 0,8 à 1 ml d'Affigel-calmoduline[R] et on agite lentement le mélange à 4°C pendant 18 heures. Plus des 2/3 de l'activité enzymatique sont retenus sur le gel.

On fait sédimenter l'Affigel-calmoduline par centrifugation à 300 x g pendant 1 minute, on lave à plusieurs reprises avec NaCl 0,5 M dans le tampon A. On récupère l'adényl cyclase à partir du gel à l'aide de 2,5 ml d'urée 8,8 M dans le tampon A. On élimine l'urée par filtration sur une colonne de Séphadex G-25[R], équilibrée avec le tampon A.

Cette préparation enzymatique possède une activité spécifique de 1100 unités/mg de protéine.

On peut la conserver à -80°C sans perte d'activité sur plusieurs semaines.

## EXEMPLE 3 : PROCEDE D'OBTENTION D'ADENYL CYCLASE PURIFIEE A PARTIR D'EXTRAITS BACTERIENS

On soumet une culture de bactéries exprimant de l'AC activable par de la calmoduline à un traitement avec de l'urée 8M durant environ 2 heures à température ambiante. On récupère le surnageant qui constitue l'extrait bactérien et on le met en contact avec le gel Affigel-calmoduline dans les conditions rapportées ci-dessus.

On obtient une préparation enzymatique de pureté élevée présentant sensiblement la même activité spécifique.

**EXEMPLE 4 : ETUDE DE LA SECRETION DE AC, de PTx et de FHA, ET DE LA DL50 DE SOUCHES DE BORDETELLA**

Dans le tableau 1 ci-après, on indique les résultats obtenus en dosant les facteurs de virulence AC, PTx et FHA et en mesurant la DL50 (dose tuant 50% des souris dans un lot) de différentes souches de Bordetella.

Il s'agit de souches de B.pertussis (BP), B.parapertussis (BPP) et B.bronchiseptica (BB) correspondant soit aux souches hyperpathogènes, qui secrètent de grandes quantités d'adényl cyclase (ces souches portent la référence S dans le tableau), soit aux souches parentales des souches hyperpathogènes.

TABLEAU 1

|  | AC | PTx | FHA | DL50 |
|---|---|---|---|---|
| BP18323 | 20 | 1 | 2 | $3\ 10^8$ |
| BP18323S | 180 | 4 | 2 | $10^7$ |
| BP8144 | 2 | (+) | | |
| BP8144S | 6 | (+) | | |
| BP8132 | 1 | (+) | 32 | $3,2\ 10^8$ |
| BP8132S | 3 | (+) | 32 | $<1,2\ 10^3$ |
| BPP632 | 8 | - | 64 | $2\ 10^8$ |
| BPP632S | 35 | - | 64 | $<10^8$ |
| BB973S | 110 | - | 16 | $4\ 10^6$ |

Ces résultats mettent en évidence la production élevée d'adényl cyclase par des souches hyperpathogènes, plus spécialement par B.bronchiseptica 973S et B.pertussis 18323S.

La sécrétion de la PTx est également augmentée chez les dérivés hyperpathogènes comme le montrent les résultats relatifs à B.pertussis alors que la sécrétion de la FHA reste la même chez les deux souches.

B.bronchiseptica et B.parapertussis, qui synthétisent les mêmes facteurs que B.pertussis à l'exception de la PTx, induisent chez la souris une alvéolite oedémateuse hémorragique semblable à celle induite par B.pertussis. En revanche, une souche dérivée de la souche virulente B.pertussis 8132, la souche B.P.348 et hébergeant un transposon Tn5 dans le gène de structure de l'AC, inactivant celui-ci, est incapable d'induire une AOHA chez la souris. Ce mutant sécrète, cependant, tous les autres facteurs de virulence, en particulier la PTx.

L'ensemble de ces résultats désigne l'AC comme facteur responsable de l'alvéolite oedémateuse hémorragique chez la souris.

**EXEMPLE 5 :**

**1. EXPERIENCES DE PROTECTION ACTIVE A PARTIR DE VACCINS BACTERIENS**

Des expériences de protection (contre B.pertussis) ont été réalisées à l'aide de différents vaccins bactériens (généralement 3 injections sous-cutanées de 250$\mu$l de suspension bactérienne, contenant $10^9$ bactéries/ml, chauffées à 56°C, à une semaine d'intervalle).

On rapporte dans le tableau 2 ci-après les résultats obtenus en utilisant pour l'élaboration des vaccins bactériens des souches de B.bronchispetica 9.73S ainsi que des souches de B.pertussis 18323S, B.parapertussis 63.25 et B.avium (Blike Hewouet).

La souche de B.pertussis est administrée deux semaines après la dernière immunisation : on injecte 50$\mu$l par voie intranasale de $10^8$ bactéries de la souche de B.pertussis 18323S vivantes.

| SOUCHE INJECTEE | EPREUVE LETALE (B.pertussis) |
|---|---|
| 0 | 10/10 |
| B.bronchiseptica | 0/10 |
| B.pertussis | 0/10 |
| B.parapertussis | 3/10 |
| B.avium | 9/10 |

On constate que les souris vaccinées à l'aide de préparations de B.bronchiseptica sont totalement protégées contre une épreuve létale de B.pertussis, (protection croisée), comme celles vaccinées avec B.pertussis.

En revanche, les souris vaccinées avec des préparations de B.avium ne sont pas protégées, ce qui joue en faveur du rôle de l'AC dans l'induction de l'alvéolite oedémateuse hémorragique.

## 2. EXPERIENCES DE PROTECTION ACTIVE A PARTIR D'ANTIGENES PURIFIES

On utilise des antigènes purifiés.

Les préparations d'AC sont purifiées, soit à partir du surnageant de culture (70% de l'enzyme sont sécrétés par la bactérie), soit à partir des bactéries en opérant avantageusement selon le procédé de la demande de brevet FR 2606789 du 17.11.86.

Des souris ont été immunisées avec différentes doses d'antigène (3 injections sous-cutanées à une semaine d'intervalle, de $250\mu$l d'antigène dans du tampon tris 10mM pH7 contenant 1mg/ml d'Am + + +) avant de faire l'épreuve létale de B.pertussis.

Pour l'épreuve létale, on injecte par voie intranasale $50\mu$l de suspension bactérienne de la souche virulente et hyperpathogène 18323S, 2 semaines après la dernière immunisation. La concentration en bactéries de la suspension est de $10^8$ dans l'essai a, $2 \times 10^8$ dans l'essai b, et $10^7$ dans l'essai c.

Dans le tableau 3 ci-dessous, on a rapporté les résultats de protection obtenus contre une souche de B.pertussis (BP 18323S) à différentes concentrations, après injection d'AC, d'AC recombinante d'un vaccin, de PTx et de FHA.

L'AC recombinante est avantageusement telle que décrite dans la demande de brevet FR 2621597 du 24.7.87. Il s'agit d'un fragment correspondant au gène de structure de l'AC qui a pu être exprimé dans E.coli. Ce fragment qui porte l'activité adényl cyclase et le site de fixation de la calmoduline a été purifié à homogénéité (voir figure unique).

| AC | ACrec | VACCIN | PTx | FHA | O |
|---|---|---|---|---|---|
| **a : épreuve létale B.P.18323S 10/DL50** | | | | | |
| 3/0.9µg | 3/1.5µg | 3/10$^8$ | 2/2µg | 2/10µg | |
| 9/12 | 1/10 | 0/40 | 9/22 | 20/22 | 20/22 |
| **b : épreuve létale B.P.18323S 20/DL50** | | | | | |
| 3/2µg | | 3/10$^8$ | | | |
| 5/10 | | 3/10 | | | 10/10 |
| **c : épreuve létale B.P.18323S 1/DL50** | | | | | |
| 3/6µg | 3/6µg | 3/18µg | 3/2µg | 3/10µg | |
| 0/10 | 0/10 | 0/10 | 0/10 | 5/10 | 5/10 |

Les résultats de létalité sont exprimés en nombre de souris mortes sur le nombre de souris totales

Les résultats obtenus montrent l'obtention d'une synthèse d'anticorps antiAC et une protection des souris, après immunisation avec de l'AC (trois immunisations de 1,5µl d'AC). Cette protection est quasi totale lorsque l'épreuve létale est réalisée avec une quantité de bactéries égale ($10^7$ bactéries vivantes) ou 10 fois supérieure à la dose létale 50 ($10^8$ bactéries vivantes) ; elle est de 60% lorsque l'épreuve létale est réalisée avec une quantité de bactéries égale à 20 fois la dose létale 50 ($2.10^8$ bactéries létales).

Une protection totale a été obtenue après immunisation de souris avec l'AC recombinante, lorsque l'épreuve létale est réalisée avec une quantité de bactéries égale à la dose létale 50. Les immunisations réalisées avec de la FHA n'ont pas montré d'effet protecteur de cette toxine malgré la présence d'anticorps chez les souris immunisées et ceci même lorsque l'épreuve létale est réalisée avec une quantité faible de bactéries.

Les expériences réalisées avec la PTx montre une protection partielle après deux immunisations avec 2µg de PTx lorsque l'épreuve létale est 10 fois supérieure à la dose létale 50 mais il faut préciser que, dans ce cas, la synthèse d'anticorps antiPTx est très faible. Lorsque les souris sont immunisées avec 3 fois 2µg de PTx, la synthèse d'anticorps est plus importante et nous obtenons une protection totale lorsque l'épreuve létale est réalisée avec une quantité de bactéries égale à la dose létale 50.

D'après l'ensemble de ces résultats, l'adényl cyclase constitue la cytotoxine responsable de l'alvéolite oedémateuse hémorragique observée chez la souris infectée par B.pertussis et constitue un antigène protecteur contre ces lésions. La PTx et la FHA ne joueraient aucun rôle dans l'induction de cette AOHA mais pourraient être des antigènes protecteurs de ces lésions en inhibant l'adhésion des bactéries sur l'épithélium respiratoire.

**Revendications**

1. Utilisation d'une adényl cyclase provenant de Bordetella pertussis ou de Bordetella bronchiseptica ou d'un fragment actif de cette adényl cyclase, pour l'obtention d'un médicament destiné à conférer chez l'homme ou chez l'animal, une protection croisée contre les infections et les effets toxiques causés par B. bronchiseptica ou par B. pertussis, respectivement.

2. Utilisation selon la revendication 1 caractérisée en ce que l'adényl cyclase est telle qu'obtenue par mise en contact du surnageant d'une culture bactérienne de B. pertussis ou de B. bronchisseptica avec un gel Affigel Calmoduline®.

**3.** Utilisation selon la revendication 1 ou 2, caractérisée en ce que l'adényl cyclase provient de B. bronchiseptica, le médicament conférant une protection croisée contre les infections et les effets toxiques causés par B. pertussis.

**4.** Utilisation selon la revendication 3 caractérisée en ce que l'adényl cyclase provient de B. bronchiseptica souche 9.73 (CNCM I-858).

**5.** Utilisation selon la revendication 1 ou 2 caractérisée en ce que l'adényl cyclase provient de B. pertussis, le médicament conférant une protection croisée contre les infections et les effets toxiques causés par B. bronchiseptica.

**6.** Utilisation selon la revendication 1 caractérisée en ce que l'adényl cyclase comporte au moins la partie active de la séquence d'acides aminés représentée sur la figure, particulièrement le fragment $Gly^{361}$ - $Ser^{689}$.

**7.** Utilisation selon l'une quelconque des revendications précédentes caractérisée en ce que l'adényl cyclase est associée dans le même inoculum ou non avec de la FHA et/ou de la PTx.

**8.** B. bronchiseptica souche 9.73 (CNCM I-858).

**Claims**

**1.** Use of an adenylate cyclase derived from Bordetella pertussis or Bordetella bronchiseptica or an active fragment of this adenylate cyclase for the production of a medicine designed to confer on man and animals a cross-protection against the infections and toxic effects caused by B. bronchiseptica or by B. pertussis, respectively.

**2.** Use according to Claim 1, characterized in that the adenylate cyclase is that obtained by placing the supernatant of a bacterial culture of B. pertussis or B. bronchiseptica in contact with an Affigel Calmoduline$^R$ gel.

**3.** Use according to Claim 1 or 2, characterized in that the adenylate cyclase is derived from B. bronchiseptica, the medicine conferring a cross-protection against the infections and toxic effects caused by B. pertussis.

**4.** Use according to Claim 3, characterized in that the adenylate cyclase is derived from B. bronchiseptica strain 9.73 (CNCM 1-858).

**5.** Use according to Claim 1 or 2, characterized in that the adenylate cyclase is derived from B. pertussis, the medicine conferring a cross-protection against the infections and toxic effects caused by B. bronchiseptica.

**6.** Use according to Claim 1, characterized in that the adenylate cyclase contains at least the active part of the amino acid sequence shown in the figure, particularly the fragment $Gly^{361}$ - $Ser^{689}$.

**7.** Use according to any one of the preceding Claims, characterized in that the adenylate cyclase is combined in the same inoculum or not the same with FHA and/or PTx.

**8.** B. bronchiseptica strain 9.73 (CNCM I-858).

**Patentansprüche**

**1.** Verwendung einer Adenylcyclase aus Bordetella pertussis oder Bordetella bronchiseptica oder eines wirksamen Fragments dieser Adenylcyclase zur Herstellung eines Arzneimittels, welches dazu bestimmt ist, Mensch oder Tier einen Kreuzschutz zu verleihen gegen Infektionen und toxische Wirkungen, welche durch B. bronchiseptica bzw. B. pertussis verursacht werden.

**2.** Verwendung nach Anspruch 1,
dadurch gekennzeichnet,
daß die Adenylcyclase erhalten wird durch Inkontaktbringen eines Überstandes einer Bakterienkultur von B. pertussis oder B. bronchiseptica mit einem Gel Affigel Calmoduline$^{(R)}$.

**3.** Verwendung nach Anspruch 1 oder 2,
dadurch gekennzeichnet,
daß die Adenylcyclase aus B. bronchiseptica stammt, wobei das Arzneimittel einen Kreuzschutz gegen Infektionen und toxische Wirkungen, welche von B. pertussis verursacht werden, verleiht.

**4.** Verwendung nach Anspruch 3,
dadurch gekennzeichnet,
daß die Adenylcyclase aus B. bronchiseptica Stamm 9.73 (CNCM I-858) stammt.

**5.** Verwendung nach Anspruch 1 oder 2,
dadurch gekennzeichnet,
daß die Adenylcyclase aus B. pertussis stammt, wobei das Arzneimittel einen Kreuzschutz gegen Infektionen und toxische Wirkungen, welche von B. bronchiseptica verursacht werden, verleiht.

**6.** Verwendung nach Anspruch 1,
dadurch gekennzeichnet,
daß die Adenylcyclase mindestens den aktiven Bereich der in der Figur dargestellten Aminosäuresequenz, insbesondere das Fragment Gly$^{361}$ - Ser$^{689}$, umfaßt.

**7.** Verwendung nach irgendeinem der vorangegangenen Ansprüche,
dadurch gekennzeichnet,
daß die Adenylcyclase in demselben Inokulum zusammen mit FHA und/oder PTx vorliegt oder nicht.

**8.** B. bronchiseptica Stamm 9.73 (CNCM I-858).

I a

GAAGCCTTGTTCTTCTTTTCATTAGAAAGAAATATGCGCTTTGTGTTTAGGATGATTTTC

    .        .        .        .        .      -200

CTGTCCGAGTAGGGTGGATCCAAATTTTCCGGATTGGTGGGAATTTGTGCATTTTCACTG

    .        .        .        .        .        .

CGAATGTTGGAATAATTTCGCCCATCGTCATACGACATGCTGGATGTTTGGTTCTTGCAG

    .        .        .      -100        .        .

AAGGATGAGGTTCTGAGCGCTACACACCGGTTGCGTCGGTGCGAATCCGTTCAATCGACT

    .        .        .        .        .        .

                    MetGlnGlnSerHisGlnAlaGlyTyrAlaAsnAlaAlaAsp

ACTTATCGACAGATCCACATGCAGCAATCGCATCAGGCTGGTTACGCAAACGCCGCCGAC

    .        0         .        .        .

ArgGluSerGlyIleProAlaAlaValLeuAspGlyIleLysAlaValAlaLysGluLys

CGGGAGTCTGGCATCCCCGCAGCCGTACTCGATGGCATCAAGGCCGTGGCGAAGGAAAAA

    .        .        .        .        .      100

AsnAlaThrLeuMetPheArgLeuValAsnProHisSerThrSerLeuIleAlaGluGly

AACGCCACATTGATGTTCCGCCTGGTCAACCCCCATTCCACCAGCCTGATTGCCGAAGGG

    .        .        .        .        .        .

ValAlaThrLysGlyLeuGlyValHisAlaLysSerSerAspTrpGlyLeuGlnAlaGly

GTGGCCACCAAAGGATTGGGCGTGCACGCCAAGTCGTCCGATTGGGGGTTGCAGGCGGGC

    .        .        .      200        .        .

TyrIleProValAsnProAsnLeuSerLysLeuPheGlyArgAlaProGluValIleAla

TACATTCCCGTCAACCCGAATCTTTCCAAACTGTTCGGCCGTGCGCCCGAGGTGATCGCG

    .        .        .        .        .        .

Ib

ArgAlaAspAsnAspValAsnSerSerLeuAlaHisGlyHisThrAlaValAspLeuThr

CGGGCCGACAACGACGTCAACAGCAGCCTGGCGCATGGCCATACCGCGGTCGACCTGACG

    .      300        .        .        .        .

LeuSerLysGluArgLeuAspTyrLeuArgGlnAlaGlyLeuValThrGlyMetAlaAsp

CTGTCGAAAGAGCGGCTTGACTATCTGCGGCAAGCGGGCCTGGTCACCGGCATGGCCGAT

    .        .        .        .        .      400

GlyValValAlaSerAsnHisAlaGlyTyrGluGlnPheGluPheArgValLysGluThr

GGCGTGGTCGCGAGCAACCACGCAGGCTACGAGCAGTTCGAGTTTCGCGTGAAGGAAACC

    .        .        .        .        .        .

SerAspGlyArgTyrAlaValGlnTyrArgArgLysGlyGlyAspAspPheGluAlaVal

TCGGACGGGCGCTATGCCGTGCAGTATCGCCGCAAGGGCGGCGACGATTTCGAGGCGGTC

    .        .        .      500        .        .

LysValIleGlyAsnAlaAlaGlyIleProLeuThrAlaAspIleAspMetPheAlaIle

AAGGTGATCGGCAATGCCGCCGGTATTCCACTGACGGCGGATATCGACATGTTCGCCATT

    .        .        .        .        .        .

MetProHisLeuSerAsnPheArgAspSerAlaArgSerSerValThrSerGlyAspSer

ATGCCGCATCTGTCCAACTTCCGCGACTCGGCGCGCAGTTCGGTGACCAGCGGCGATTCG

    .      600      .        .        .    . .        .

ValThrAspTyrLeuAlaArgThrArgArgAlaAlaSerGluAlaThrGlyGlyLeuAsp

GTGACCGATTACCTGGCGCGCACGCGGCGGGCCGCCAGCGAGGCCACGGGCGGCCTGGAT

    .        .        .        .        .      700

ArgGluArgIleAspLeuLeuTrpLysIleAlaArgAlaGlyAlaArgSerAlaValGly

CGCGAACGCATCGACTTGTTGTGGAAAATCGCTCGCGCCGGCGCCCGTTCCGCAGTGGGC

    .        .        .        .        .        .

1c

ThrGluAlaArgArgGlnPheArgTyrAspGlyAspMetAsnIleGlyValIleThrAsp
ACCGAGGCGCGTCGCCAGTTCCGCTACGACGGCGACATGAATATCGGCGTGATCACCGAT
　　　　　　·　　　　　　·　　　　　　·　　　　　800　　　　　　·　　　　　　·

PheGluLeuGluValArgAsnAlaLeuAsnArgArgAlaHisAlaValGlyAlaGlnAsp
TTCGAGCTGGAAGTGCGCAATGCGCTGAACAGGCGGGCGCACGCCGTCGGCGCGCAGGAC
　　　　　·　　　　　·　　　　　·　　　　　·　　　　　·　　　　　·

ValValGlnHIsGlyThrGluGlnAsnAsnProPheProGluAlaAspGluLysIlePhe
GTGGTCCAGCATGGCACTGAGCAGAACAATCCTTTCCCGGAGGCAGATGAGAAGATTTTC
　　900　　　　　·　　　　　·　　　　　·　　　　　·　　　　　·

ValValSerAlaThrGlyGluSerGlnMetLeuThrARgGlyGlnLeuLysGluTyrIle
GTCGTATCGGCCACCGGTGAAAGCCAGATGCTCACGCGCGGGCAACTGAAGGAATACATT
　　　　　·　　　　　·　　　　　·　　　　　·　　　　　·　　　　1000

GlyGlnGlnArgGlyGluGlyTyrValPheTyrGluAsnArgAlaTyrGlyValAlaGly
GGCCAGCAGCGCGGCGAGGGCTATGTCTTCTACGAGAACCGTGCATACGGCGTGGCGGGG
　　　　　·　　　　　·　　　　　·　　　　　·　　　　　·　　　　　·

LysSerLeuPheAspAspGlyLeuGlyAlaAlaProGlyValProSerGlyArgSerLys
AAAAGCCTGTTCGACGATGGGCTGGGAGCCGCGCCCGGCGTGCCGAGCGGACGTTCGAAG
　　　　　·　　　　　·　　　　　·　　　　1100　　　　　·

PheSerProAspValLeuGluThrValProAlaSerProGlyLeuArgArgProSerLeu
TTCTCGCCGGATGTACTGGAAACGGTGCCGGCGTCACCCGGATTGCGGCGGCCGTCGCTG
　　　　　·　　　　　·　　　　　·　　　　　·　　　　　·　　　　　·

GlyAlaValGluArgGlnAspSerGlyTyrAspSerLeuAspGlyValGlySerArgSer
GGCGCAGTGGAACGCCAGGATTCCGGCTATGACAGCCTTGATGGGGTGGGATCGCGATCG
　　　　　·　　　　1200　　　　　·　　　　　·　　　　　·　　　　　·

13

1d

PheSerLeuGlyGluValSerAspMetAlaAlaValGluAlaAlaGluLeuGluMetThr

TTCTCGTTGGGCGAGGTGTCCGACATGGCCGCCGTGGAAGCGGCGGAACTGGAAATGACC

    •        •        •        •        •     1300

ArgGlnValLeuHisAlaGlyAlaArgGlnAspAspAlaGluProGlyValSerGlyAla

CGGCAAGTCTTGCACGCCGGGGCGCGGCAGGACGATGCCGAGCCGGGCGTGAGCGGTGCG

    •        •        •        •        •        •

SErAlaHisTrpGlyGlnArgAlaLeuGlnGlyAlaGlnAlaValAlaAlaAlaGlnArg

TCGGCGCACTGGGGGCAGCGGGCGCTGCAGGGCGCCCAGGCGGTGGCGGCGGCGCAGCGG

    •        •        •     1400        •        •

LeuValHisAlaIleAlaLeuMetThrGlnPheGlyArgAlaGlySerThrAsnThrPro

CTGGTTCATGCCATTGCCCTGATGACGCAATTCGGCCGGGCCGGTTCCACCAACACGCCG

    •        •        •        •        •        •

GlnGluAlaAlaSerLeuSerAlaAlaValPheGlyLeuGlyGluAlaSerSerAlaVal

CAGGAAGCGGCCTCGTTGTCGGCGGCCGTGTTCGGCTTGGGCGAGGCCAGCAGCGCCGTG

    •    1500        •        •        •        •

AlaGluThrValSerGlyPhePheArgGlySerSerArgTrpAlaGlyGlyPheGlyVal

GCCGAAACCGTGAGCGGTTTTTTCCGCGGGTCTTCGCGCTGGGCCGGCGGTTTCGGCGTG

    •        •        •        •        •     1600

AlaGlyGlyAlaMetAlaLeuGlyGly[lyIleAlaAlaAlaValGlyAlaGlyMet Ser

GCTGGCGGCGCGATGGCGCTGGGAGGCGGCATCGCCGCGGCCGTTGGCGCCGGGATGTCG

    •        •        •        •        •        •

LeuThrAspAspAlaProAlaGlyGlnLqsAlaAlaAlaGlyAlaGluIleAlaLeuGln

TTGACCGATGACGCGCCGGCCGGACAGAAGGCCGCCGCCGGCGCCGAGATCGCGCTGCAG

    •        •        •     1700        •        •

14

le

LeuThrGlyGlyThrValGluLEuAlaSerSerIleAlaLeuAlaLeuAlaAlaAlaArg

TTGACAGGTGGAACGGTCGAGCTGGCTTCTTCCATCGCGTTGGCGCTGGCCGCGGCGCGC

    .        .        .        .        .        .

GlyValThrSerGlyLeuGlnValAlaGlyAlaSerAlaGlyAlaAlaAlaGlyAlaLeu

GGCGTGACCAGCGGCTTGCAGGTGGCCGGGGCGTCGGCCGGGGCGGCTGCCGGCGCATTG

    .    1800       .        .        .        .

AlaAlaAlaLeuSerProMetGluIleTyrGlyLKuValGlnGlnSerHisTyrAlaAsp

GCCGCGGCGCTCAGTCCCATGGAGATCTACGGCCTGGTGCAGCAATCGCACTATGCGGAT

    .        .        .        .        .    1900

GlnLeuAspLysLeuAlaGlnGluSerSerAlaTyrGlyTyrGluGlyAspAlaLeuLeu

CAGCTGGACAAGCTGGCGCAGGAATCGAGCGCATACGGTTACGAGGGCGACGCCTTGCTG

    .        .        .        .        .        .

AlaGlnLeuTyrArgAspLysThrAlaAlaGluGlyAlaValAlaGlyValSerAlaVal

GCCCAGCTGTATCGCGACAAGACGGCCGCCGAGGGCGCCGTCGCCGGCGTCTCCGCCGTC

    .        .        .    2000      .        .

LeuSerThrValGlyAlaAlaValSerIleAlaAlaAlaAlaSerValValGlyAlaPro

CTGAGCACGGTGGGGGCGGCGGTGTCGATCGCCGCGGCGGCCAGCGTGGTAGGGGCCCCG

    .        .        .        .        .        .

ValAlaValValThrSerLeuLeuThrGlyAlaLeuAsnGlyIleLeuArgGlyValGln

GTGGCGGTGGTCACTTCCTTGCTGACCGGGGCTCTCAACGGCATCCTGCGCGGCGTGCAG

    .    2100       .        .        .        .

GlnProIleIle[luLysLeuAlaAsnAspTyrAlaArgLysIleAspGluLeuGlyGly

CAGCCCATCATCGAAAAGCTGGCCAACGATTACGCTCGCAAGATCGACGAGCTGGGCGGG

    .        .        .        .        .    2200

If

ProGlnAlaTyrPheGluLysAsnLeuGlnAlaArgHisGluGlnLeuAlaAsnSerAsp
CCGCAAGCGTACTTCGAGAAAAACCTGCAGGCGCGTCACGAACAACTGGCCAATTCGGAC
    .       .       .       .       .       .

GlyLeuArgLysMetLeuAlaAspLeuGlnAlaGlyTrpAsnAlaSerSerValIleGly
GGCCTACGGAAAATGCTGGCCGACCTGCAGGCCGGTTGGAACGCCAGCAGCGTGATCGGG
    .       .       .     2300     .       .

ValGlnThrThrGluIleSerLysSerAlaLUuGluLeuAlaAlaIleThrGlyAsnAla
GTGCAGACGACAGAGATCTCCAAGTCGGCGCTCGAACTGGCCGCCATTACCGGCAACGCG
    .       .       .       .       .       .

AspAsnLeuLysSerValAspValPheValAspArgPheValGlnGlyGluArgValAla
GACAACCTGAAATCCGTCGACGTGTTCGTGGACCGCTTCGTCCAGGGCGAGCGGGTGGCC
    .    2400     .       .       .       .

GlyGlnProValValLeuAs-ValAlaAlaGlyGlyIleAspIleAlaSerArgLysGly
GGCCAGCCGGTGGTCCTCGACGTCGCCGCCGGCGGCATCGATATCGCCAGCCGCAAGGGC
    .       .       .       .       .     2500

GluArgProAlaLeuThrPneIleThrProLeuAlaAlaProGlyGluGluGlnArgArg
GAGCGGCCGGCGCTGACGTTCATCACGCCGCTGGCCGCGCCAGGAGAAGAGCAGCGCCGG
    .       .       .       .       .       .

ARgThrLysThrGlyLysSerGluPheThrThrPheValGluIleValGlyLysGlnAsp
CGCACGAAAACGGGCAAGAGCGAATTCACCACATTCGTCGAGATCGTGGGCAAGCAGGAC
    .       .       .     2600     .       .

ArgTrpArgIleArgAspGlyAlaAlaAspThrThrIleAspLeuAlaLysValValSer
CGCTGGCGCATCCGGGACGGCGCGGCCGACACCACCATCGATCTGGCCAAGGTGGTGTCG
    .       .       .       .       .       .

1g

GlnLeuValAspAlaAsnGlyValLeuLysHisSerIleLysLeuAspValIleGlyGly
CAACTGGTCGACGCCAATGGCGTGCTCAAGCACAGCATCAAACTGGATGTGATCGGCGGA

       .     2700     .       .       .       .

AspGlyAspAspValValLeuAlaAsnAlaSerArgIleHisTyrAspGlyGlyAlaGly
GATGGCGATGACGTCGTGCTTGCCAATGCTTCGCGCATCCATTATGACGGCGGCGCGGGC

       .       .       .       .       .    2800

ThrAsnThrValSerTyrAlaAlaLeuGlyArgGlnAspSerIleThrValSerAlaAsp
ACCAACACGGTCAGCTATGCCGCCCTGGGTCGACAGGATTCCATTACCGTGTCCGCCGAC

       .       .       .       .       .       .

GlyGluArgPheAsnValArgLysGlnLeuAsnAsnAlaAsnValTyrArgGluGlyVal
GGGGAACGTTTCAACGTGCGCAAGCAGTTGAACAACGCCAACGTGTATCGCGAAGGCGTG

       .       .       .    2900     .       .

AlaThrGlnThrThrAlaTyrGlyLysArgThrGluAsnValGlnTyrArgHisValGlu
GCTACCCAGACAACCGCCTACGGCAAGCGCACGGAGAATGTCCAATACCGCCATGTCGAG

       .       .       .       .       .       .

LeuAlaArgValGlyGlnValValGluValAspThrLeuGluHisValGlnHisIleIle
CTGGCCCGTGTCGGGCAAGTGGTGGAGGTCGACACGCTCGAGCATGTGCAGCACATCATC

       .     3000     .       .       .       .

GlyGlyalaGlyAsnAspSerIleThrGlyAsnAlaHisAspAsnPheLeuAlaGlyGly
GGCGGGGCCGGCAACGATTCGATCACCGGCAATGCGCACGACAACTTCCTAGCCGGCGGG

       .       .       .       .       .    3100

SerGlyAspAspArgLeuAspGlyGlyAlaGlyAsnAspThrLeuValGlyGlyGluGly
TCGGGCGACGACAGGCTGGATGGCGGCGCCGGCAACGACACCCTGGTTGGCGGCGAGGGC

       .       .       .       .       .       .

17

1h

GlnAsnThrValIleGlyGlyAlaGlyAspAspValPheLeuGlnAspLeuGlyValTrp
CAAAACACGGTCATCGGCGGCGCCGGCGACGACGTATTCCTGCAGGACCTGGGGGTATGG
     .      .      .    3200    .      .

SerAsnGlnLeuAspGlyGlyAlaGlyValAspThrValLysTyrAsnValHisGlnPro
AGCAACCAGCTCGATGGCGGCGCGGGCGTCGATACCGTGAAGTACAACGTGCACCAGCCT
     .      .      .      .      .      .

SerGluGluArgLeuGluArgMetGlyAspThrGlyIleHisAlaAspLeuGlnLysGly
TCCGAGGAGCGCCTCGAACGCATGGGCGACACGGGCATCCATGCCGATCTTCAAAAGGGC
     .    3300    .      .      .      .

ThrValGluLysTrpProAlaLeuAsnLeuPheSerValAspHisValLysAsnIleGlu
ACGGTCGAGAAGTGGCCGGCCCTGAACCTGTTCAGCGTCGACCATGTCAAGAATATCGAG
     .      .      .      .      .    3400

AsnLeuHisGlySerArgLeuAsnAspArgIleAlaGlyAspAspGlnAspAsnGluLeu
AATCTGCACGGCTCCCGCCTAAACGACCGCATCGCCGGCGACGACCAGGACAACGAGCTC
     .      .      .      .      .      .

TrpGlyHisAspGlyAsnAspThrIleArgGlyArgGlyGlyAspAspIleLeuArgGly
TGGGGCCACGATGGCAACGACACGATACGCGGCCGGGGCGGCGACGACATCCTGCGCGGC
     .      .      .    3500    .      .

GlyLeuGlyLeuAspThrLeuTyrGlyGluAspGlyAsnAspIlePheLeuGlnAspAsp
GGCCTGGGCCTGGACACGCTGTATGGCGAGGACGGCAACGACATGTTCCTGCAGGACGAC
     .      .      .      .      .      .

GluThrValSerAspAspIleAspGlyGlyAlsGlyLeuAspThrValAspTyrSerAla
GAGACCGTCAGCGATGACATCGACGGCGGCGCGGGGCTGGACACCGTCGACTACTCCGCC
     .    3600    .      .      .      .

li

MetIleHisProGlyArgIleValAlaProHisGluTyrGlyPheGlyIleGluAlaAsp
ATGATCCATCCAGGCAGGATCGTTGCGCCGCATGAATACGGCTTCGGGATCGAGGCGGAC
     .       .       .       .       .     3700

LeuSerArgGluTrpValArgLysAlaSerAlaLeuGlyValAspTyrTyrAspAsnVal
CTGTCCAGGGAATGGGTGCGCAAGGCGTCCGCGCTGGGCGTGGACTATTACGATAATGTC
     .       .       .       .       .       .

ArgAsnValGluAsnValIleGlyThrSerMetLysAspValLeuTleGlyAspAlaGln
CGCAATGTCGAAAACGTCATCGGTACGAGCATGAAGGATGTGCTCATCGGCGACGCGCAA
     .       .       .     3800       .       .

AlaAsnThrLeuMetGlyGlnGlyGlyAspAspThrValArgGlyGlyAspGlyAspAsp
GCCAATACCCTGATGGGCCAGGGCGGCGACGATACCGTGCGCGGCGGCGACGGCGATGAT
     .       .       .       .       .       .

LeuLeuP æGly-lyAspGlyAsnAspMetLeuTyrGlyAspAlaGlyAsnAspThrLeu
CTGCTGTTCGGCGGCGACGGCAACGACATGCTGTATGGCGACGCCGGCAACGACACCCTC
     .    3900       .       .       .       .

TyrGlyGlyLeuGlyAspAspThrLeuGluGlyGlyAlaGlyAsnAspTrpPheGlyGly
TACGGGGGGCTGGGCGACGATACCCTTGAAGGCGGCGCGGGCAACGATTGGTTCGGCCAG
     .       .       .       .       .     4000

ThrGlnAlaArgGluHisAspValLeuArgGlyGlyAspGlyValAspThrValAspTyr
ACGCAGGCGCGCGAGGATGACGTGCTGCGCGGCGGAGATGGGGTGGATACCGTCGATTAC
     .       .       .       .       .       .

SerGlnThrGlyAlaHisAlaGlyIleAlaAlaGlyArgIleGlyLeuGlyIleLeuAla
AGCCAGACCGGCGCGCATGCCGGCATTGCCGCGGGTCGCATCGGGCTGGGCATCCTGGCT
     .       .       .     4100       .       .

1 j

ApLeuGlyAlaGlyArgValAspLysLeuGlyGluAlaGlySerSerAlaTyrAspThr

GACCTGGGCGCCGGCCGCGTCGACAAGCTGGGCGAGGCCGGCAGCAGCGCCTACGATACG

ValSerGlyIleGluAsnValValGlyThrGluLeuAlaAspArgIleThrGlyAspAla

GTTTCCGGTATCGAGAACGTGGTGGGCACGGAACTGGCCGACCGCATCACGGGCGATGCG

4200

GlnAlaAsnValLeuArgGlyAlaGlyGlyAlaAspValLeuAlaGlyGlyGluGlyAsp

CAGGCCAACGTGCTGCGCGGCGCGGGTGGCGCCGACGTGCTTGCGGGCGGCGAGGGCGAC

4300

AspValLeuLeuGlyGlyAspGlyAspAspGlnLeuSerGlyAspAlaGlyArgAspArg

GATGTGCTGCTGGGCGGCGACGGCGACGACCAGCTGTCGGGCGACGCCGGACGCGATCGC

LeuTyrGlyGluAlaGlyAspAspTrpPhePheGlnAspAlaAlaAsnAlaGlyAsnLeu

TTGTACGGCGAAGCCGGTGACGACTGGTTCTTCCAGGATGCCGCCAATGCCGGCAATCTG

4400

LeuAspGlyGlyAspGlyArgAspThrValAspPheSerGlyProGlyArgGlyLeuAsp

CTCGACGGCGGCGACGGCCGCGATACCGTGGATTTCAGCGGCCCCGGGCCGGGGCCTCGAC

AlaGlyAlaLysGlyValPheLeuSerLeuGlyLysGlyPheAlaSerLeuMetAspGlu

GCCGGCGCAAAGGGCGTATTCCTGAGCTTGGGCAAGGGGTTCGCCAGCCTGATGGACGAA

4500

ProGlu ThrSerAsnValLeuArgAsnIleGluAsnAlaValGlySerAlaArgAspAsp

CCCGAAACCAGCAACGTGTTGCGCAATATCGAGAACGCCGTGGGCAGCGCGCGTGATGAC

4600

lk

ValLeuIleGlyAspAlaGlyAlaAsnValLeuAsnGlyLeuAlaGlyAsnAspValLeu

GTGCTGATCGGCGACGCAGGCGCCAACGTCCTCAATGGCCTGGCGGGCAACGACGTGCTG

      .      .      .      .      .      .

SerGlyGlyAlaGlyAspAspValLeuLeuGlyAspGlullySerAspLeuLeuSerGly

TCCGGCGGCGCTGGCGACGATGTGCTGCTGGGCGACGAGGGCTCGGACCTGCTCAGCGGC

      .      .      .     4700      .      .

AspAlaGlyAsnAspAspLeuPheGlyglyGlnGlyAspA þThrTyrLeuPheGlyVal

GATGCGGGCAACGACGATCTGTTCGGCGGGCAGGGCGATGATACTTATCTGTTCGGGGTC

      .      .      .      .      .      .

GlyTyrGlyH;sAspThrIleTyrGluSerGlyGlyGlyHisAspThrIleArgIleAsn

GGGTACGGGCACGACACGATCTACGAATCGGGCGGCGGCCATGACACCATCCGCATCAAC

      .     4800      .      .      .      .

AlaGlyAlaAspGlnLeuTrpPxeAlaArgGlnGlyAsnAspLeuGluIleArgIleLeu

GCGGGGGCGGACCAGCTGTGGTTCGCGCGCCAGGGCAACGACCTGGAGATCCGCATTCTC

      .      .      .      .      .     4900

GlyThrAspAspAlaLeuThrValHisAspTrpTyrArgAspAlaAspHisArgValGlu

GGCACCGACGATGCACTTACCGTGCACGACTGGTATCGCGACGCCGATCACCGGGTGGAA

      .      .      .      .      .      .

IleIleHisAlaAlaAsnGlnAlaValAspGlnAlaGlyIleGluLysLeuValGluAla

ATCATCCATGCCGCCAACCAGGCGGTAGACCAGGCAGGCATCGAAAAGCTGGTCGAGGCA

      .      .      .     5000      .      .

MetAlaGlyTyrProAspProGlyAlaAlaAlaAlaAlaProProAlaAlaArgValPro

ATGGCGCAGTATCCGGACCCCGGCGCGGCGGCGGCTGCCCCGCCGGCGGCGCGCGTGCCG

      .      .      .      .      .      .

11

AspThrLeuMetGlnSerLeuAlaValAsnTrpArg***

GACACGCTGATGCAGTCCCTGGCTGTCAACTGGCGCTGAAGCGCCGTGAATCACGGCCCG

.          5100              .              .              .              .

CCTGCCTCGCGCGGCGGCGCCGTCTCTTTGCGTTCTTCTCCGAGGTATTTCCCATCATGA

.              .              .              .              .          5200

CGTCGCCCGCGGCGCAATGCGCCAGCGTGCCCGATTCCGGGTTGCTCTGCCTGGTCATGC

.              .              .              .              .              .

TGGCTCGCTATCACGGATTGGCAGCCGATCCCGAGCAGTTGCGGCATGAGTTCGCCGAGC

.              .              .          5300              .              .

AGGCATTCTGTAGCGAAACGATACAGCCTGGCGGCGCGCCGGGTCGGCCTGAAAGTGCGG

.              .              .              .              .              .

CGGCACCGACCCGCGCCGGCGCGGCTGCCACGCGCGCCGCTGCCGGCCATCGCGCTGGAC

          5400              .              .              .              .

CGGCAGGGCGGCTACTTTGTT